# EUROPEAN PATENT APPLICATION

(11) **EP 4 309 568 A1**
(43) Date of publication of application: **24.01.2024**
(21) Application number: 22185694.1
(22) Date of filing: 19.07.2022
(51) Int. Cl.: A61B 5/00, A61B 5/08

(54) **METHOD AND APPARATUS FOR SPECTROSCOPIC CANCER SCREENING**

(71) Applicant: Canostix GmbH, 10713 Berlin (DE)
(72) Inventor: Kirchberg, Kristina, 12159 Berlin (DE); Pieszczek, Lukasz, 40-755 Katowice (PL); Werner, Pascal, Mebane, NC 27302 (US); Hocotz, Thaddäus, 10711 Berlin (DE)
(74) Representative: Herrmann, Daniel

(57) **Abstract**

The present invention provides a Computer-implemented method for diagnosing a disease in a subject, the method comprising:
- obtaining spectroscopic measurement data comprising a plurality of spectra of a sample of the subject;
- rejecting a spectrum from the plurality of spectra if the spectrum fulfils one or more spectrum outlier criteria;
- classifying the plurality of spectra using a plurality of classifiers to obtain a plurality of classification results; and
- determining a result indicative of whether the disease is present based on the plurality of classification results.

## Description

### TECHNICAL FIELD

The present invention relates to a Computer-implemented method for diagnosing a disease in a subject. The present invention also relates to an apparatus and a computer-readable medium.

### BACKGROUND

Spectroscopy is an exceptional analytical tool used for obtaining very specific information regarding a sample's molecular composition, with particular usefulness in the area of cancer diagnosis. During a spectroscopy measurement, a sample is illuminated by a laser at a specific wavelength that interacts with intramolecular bonds, causing them to vibrate and consecutively emit a signal. The energy difference between the incident and scattered photons is thus equivalent to the energy of numerous molecular vibrations in the sample. In this way spectroscopy can provide a "fingerprint" of the sample that is a highly specific spectroscopic characteristic of the material and the disease.

Each spectroscopic measurement typically contains several hundreds to several thousands data points. Processing this large amount of data to reliably identify a disease, in particular early stages of cancer, is a significant challenge. Thus, there is a need for an improved method for diagnosing a disease in a subject based on spectroscopic measurement data.

### SUMMARY OF THE INVENTION

The objective of the present invention is to provide a Computer-implemented method for diagnosing a disease in a subject, which overcomes one or more problems of the prior art.

A first aspect of the invention provides a Computer-implemented method for diagnosing a disease in a subject, the method comprising:
- obtaining spectroscopic measurement data comprising a plurality of spectra of a sample of the subject;
- rejecting a spectrum from the plurality of spectra if the spectrum fulfils one or more spectrum outlier criteria;
- classifying the plurality of spectra using a plurality of classifiers to obtain a plurality of classification results; and
- determining a result indicative of whether the disease is present based on the plurality of classification results.

The method of the first aspect has been shown in experiments to yield highly accurate results even if noise is present in the spectroscopic measurement data. In particular, the combination of rejecting of spectra based on spectrum outlier criteria and the plurality of classifiers that yield classification results has been shown to yield particularly reliable results.

The step of obtaining spectroscopic measurement data can comprise obtaining the data in a digital format, e.g. from a digital memory, a network, a computer-readable medium, etc.

Experiments have shown that the method of the first aspect can be used for different diseases, including different types of cancer and neurodegenerative disease such as dementia.

In a first implementation of the method according to the first aspect, the disease is a cancer, in particular a lung cancer. Experiments have shown that the method of the first aspect is particularly useful for cancer, in particular lung cancer. In particular, early stages of cancer can be detected.

In a further implementation of the method according to the first aspect, the one or more spectrum outlier criteria comprise a spike value criterion that rejects a spectrum if the spectrum comprises one or more values that exceed a median absolute variation of the spectrum. The median absolute deviation is a reliable measure of how spread out the data points of the spectrum are. Therefore, if one or more values exceed a median absolute variation of the spectrum, it can be assumed that this is a spike that is due e.g. to measurement artifacts and hence the entire spectrum can be considered unreliable.

The spike value criterion can be determined such that a predetermined number of values must exceed the median absolute variation in order for the spike value criterion to indicate that the spectrum should be rejected. For example, the number can be an absolute number such as 1, 2, 3, 5, 8 or 10. In other embodiments, the number can be a predetermined percentage of all values of the spectrum.

In a further implementation of the method according to the first aspect, the median absolute variation is calculated based on a comparison of the plurality of spectra using Chebyshev distance. Experiments have shown that the use of the Chebyshev distance yields particularly good results.

In a further implementation of the method according to the first aspect, the plurality of classifiers comprise:
- one or more disease classifiers that indicate whether the disease is present, and
- one or more alternate classifiers that indicate whether one or more alternate states are present,
wherein in particular the one or more alternate states can comprise one or more conditions, in particular one or more lung diseases, in particular chronic obstructive pulmonary disease, COPD and/or asthma.

In this implementation, the one or more individual disease classifiers can "compete" against one or more individual alternate classifiers to obtain a more reliable result whether the disease is present.

For example, a positive result indicative of that the disease is present can be determined if more disease classifiers than alternate classifiers yield a positive result.

The one or more alternate conditions can comprise alternate diseases, i.e., diseases different from the disease that the method of the first aspect is diagnosing. The one or more alternate conditions may also comprise or consist of conditions that are not diseases. For example, there may be certain genetic and/or other conditions that are considered "healthy", but may still result in spectra similar to spectra of a sample of a patient afflicted with the to-be-diagnosed disease. Thus, the alternate classifiers can be used to avoid false positives.

In a further implementation of the method according to the first aspect, the disease is diagnosed if a disease confidence value of the one or more disease classifiers is higher than an alternate confidence value of the one or more alternate classifiers.

The classifiers may be e.g. probabilistic classifiers that yield not only a positive/negative classification result, but also a confidence value on whether the positive/negative classification result is correct. The disease confidence value of the one or more classifiers can refer to an appropriate mathematical combination of the individual confidence values, e.g. an average, a mean of the individual confidence values.

In a further implementation of the method according to the first aspect, the method further comprises a step of rejecting the plurality of spectra based on one or more conditions of a class modelling technique that has been derived based on partial least square modelling of a training set of representative training spectra.

If the plurality of spectra deviate from what is expected based on the class modelling technique, it may be preferable to reject the plurality of spectra and not have a diagnosis result over obtaining an incorrect disease diagnosis. Thus, this implementation can also contribute to having only reliable results (if any).

In a further implementation of the method according to the first aspect, at least one of the plurality of classifiers is based on partial least-squares discriminant analysis. Partial least-squares discriminant analysis has been shown in experiments to be efficient to compute, yet yield accurate results.

In a further implementation of the method according to the first aspect, the plurality of classifiers comprises a plurality of staging classifiers that classify different stages of the disease against a non-cancer.

In a further implementation of the method according to the first aspect, the result indicates that the disease is present if at least one of the plurality of staging classifiers yields a positive result. In a preferred embodiment, the result indicates that the disease is present if at least one of the plurality of staging classifiers yields a positive result but the non-cancer does not yield a positive result.

In a further implementation of the method according to the first aspect, the method further comprises a step of normalizing the plurality of spectra by subtracting a mean of values of the plurality of spectra and dividing by a standard deviation of values of the plurality of spectra.

In a further implementation of the method according to the first aspect, at least one of the plurality of classifiers comprises a bagging classifier that comprises a plurality of individual classifiers.

In a further implementation of the method according to the first aspect, the plurality of classifiers comprises classifiers for at least two, preferably four, different stages of the cancer, wherein preferably a positive result indicative of that a disease is present is determined if at least one output of the classifiers for the at least two different stages of the cancer is positive. Experiments have shown that separately trained classifiers for different disease stages can yield overall more reliable results than having only one disease classifier that is trained to detect any stage of the disease.

A further aspect of the invention refers to a computer-readable storage medium storing program code, the program code comprising instructions that when executed by a processor carry out the method of the first aspect or one of the implementations of the first aspect.

### BRIEF DESCRIPTION OF THE DRAWINGS

To illustrate the technical features of embodiments of the present invention more clearly, the accompanying drawings provided for describing the embodiments are introduced briefly in the following. The accompanying drawings in the following description are merely some embodiments of the present invention, modifications on these embodiments are possible without departing from the scope of the present invention as defined in the claims.
- FIG. 1: is a flow chart of a method in accordance with an embodiment of the present disclosure,
- FIG. 2: is a flow chart of a method in accordance with a preferred embodiment of the present disclosure, and
- FIG. 3: is a flow chart of a method in accordance with an embodiment of the present disclosure.
- FIGs. 4A to 4F: illustrate five different spectroscopic measurement data, each comprising a plurality of spectra.

### DETAILED DESCRIPTION OF EMBODIMENTS

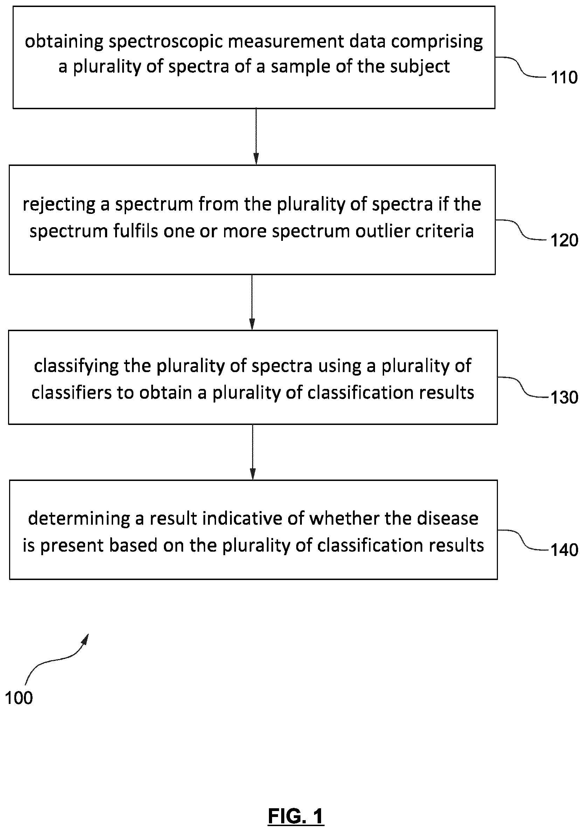

FIG. 1 is a flow chart of a method 100 for diagnosing a disease in a subject. The method may be carried out on a computer. In particular, all steps may be carried out on a computer. In particular, the method maybe carried out on a server, preferably in a cloud server.

The method comprises a first step 110 of obtaining spectroscopic measurement data comprising a plurality of spectra of a sample of the subject.

The obtaining herein may refer to reading the data from an input, e.g. a computer-readable medium, a network, a volatile memory and/or any other digital input. The spectroscopic measurement data may have been measured in a previous measurement step using suitable spectroscopic methods, e.g. using Raman spectroscopy.

The method 100 comprises a further step 120 of rejecting a spectrum from the plurality of spectra if the spectrum fulfils one or more spectrum outlier criteria. Rejecting herein may refer to removing the rejected spectrum from the plurality of spectra. The spectrum may also be rejected by marking it with a flag that indicates that this spectrum should not be used in the further processing.

A further step 130 comprises classifying the plurality of spectra using a plurality of classifiers to obtain a plurality of classification results. Each of the plurality of classifiers may have been trained using supervised machine learning techniques. In particular, neural networks, deep neural networks, Support Vector Machines and/or other trainable classification methods may be used.

In a further step 140, a result indicative of whether the disease is present based on the plurality of classification results is determined. The result may be output to a user on a user interface. The user interface may be remote from the computer where the previous steps have been carried out.

The method of FIG. 1 may use highly sensitive vibrational spectroscopy to be enabled to measure even the slightest changes in the blood plasma that are caused e.g. by the presence of a disease.

The method of FIG. 1 may comprise initial training steps that are carried out before step 110, where the method is trained using labelled data. By applying the classifiers that may have been trained on thousands of patient samples, predictive models can be built that are capable of differentiating between patients with and without disease with high accuracy.

The method may comprise two separate steps of rejecting measurements. For example, outlier detection based on a weak or oversaturated signal or a specific predetermined spectral shape can lead to the entire patient (all measurements) being excluded. Reasons can include e.g. hemolysis of the sample or strongly lipemic sample (cloudy/turbid) or any other reason resulting in a cloudy/opaque/turbid sample.

Outlier detection based on spikes: For example, 8 spectra per patient sample are collected. The individual measurements that contain spikes are rejected. The remaining spectra after spike detection can be averaged and then processed via the diagnostic classifier.

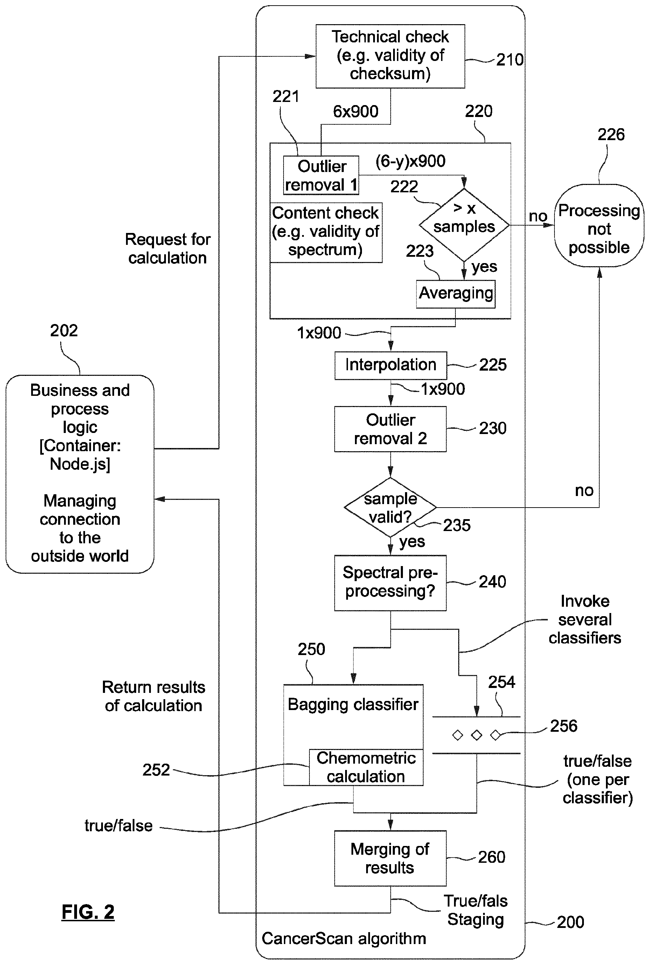

FIG. 2 is a flow chart of a further method 200 in accordance with an embodiment of the present disclosure.

Business and process logic (implemented e.g. in a container Node.js) manages connections to the outside world. In particular, a request for calculation may be issued from this business and process logic, which may initiate the method 200.

The method 200 comprises a first step 210, which involves a technical check. In particular, this may involve checking the technical correctness of the data, e.g. check of checksums if available or check that the data have the correct data types.

In a subsequent step 220, a content check of a validity of the plurality of spectra is performed. This involves a first outlier removal 221 and a second outlier removal 224.

The first outlier removal 221 is focussed on spike removal, while the second removal is focussed on overall outliers, e.g. over- or undersaturated spectra or other specific predetermined spectral shapes. For each spectrum multiple measurements (preferably between 6 and 12, e.g. 8) are provided. The content check 220 may also include a step 223 of averaging of the measurements to increase signal to noise ratio and then checking the averaged spectra.

The first outlier removal 221 may be used to detect cosmic spikes in Raman signals The algorithm may use the Chebyshev distance and median absolute deviation (MAD). Consecutive spectra from one sample (e.g. 6 or 8 spectra) are compared to each other using the Chebyshev distance. Then those values that exceed the calculated MAD are considered spikes and the corresponding spectrum may be removed.

In step 222 it is determined whether more than x samples are remaining, where x is a predetermined number. If less than x samples are remaining, no processing is possible and the method terminates in step 226. Step 226 may result in outputting a termination/failure signal that is transmitted to a user. For example, an optic and/or acoustic signal may be put out. Thus, an operator may be immediately notified that no diagnostic result could be obtained.

In step 223, an averaging of the remaining samples is performed. This is followed by an interpolation step 225. The wavenumbers (x axis) of the measurements may vary from measurement to measurement. It usually varies from spectrometer to spectrometer. By recalculating/interpolating the intensities to the wavenumbers used for the training, this can be mitigated.

In step 230, a second outlier removal, which is a general outlier removal, is used to detect outliers, such as spectra representing samples other than plasma, samples that have been measured incorrectly, and/or samples degraded/spoiled before measurement

This step 230 preferably uses the partial least squares, PLS, method and the concept of class modelling technique (CMT). First, a set of representative training spectra of blood plasma (representing different types of diseases) is selected. On the basis of the chosen training spectra, the PLS method develops logical criteria for the identification of the entire possible range of spectra that represent correctly measured plasma samples (no matter what kind of disease the sample represents). In a preferred implementation, all samples that do not meet the conditions defined by the developed CMT model are rejected as abnormal or samples of unknown origin (non-plasma sample).

In step 235, it is checked whether the sample is valid, e.g. whether the quality of the (preferably averaged) spectral signal after the second outlier removal 224 is sufficient. If not, the method terminates in step 226.

If the check in step 235 is passed, the method continues with a spectral pre-processing in step 240. Herein, SNV (standard normal variate) maybe used to process the spectrograms.

In step 250, a bagging classification is performed. Specifically, bagging (also known as ensemble learning) is applied in this step. Instead of one PLS-DA classifier (see step 260), multiple (e.g. 500) classifiers are trained and used for the prediction. Preferably, the majority of the classifiers of the plurality of classifiers determine the outcome of the overall classifier.

Step 250 includes one or more executions of step 252, where individual classifiers are computed in a chemometric calculation. In particular, these individual classifiers may be based on PLS-DA (Partial Least-Squares Discriminant Analysis), which is an extension of PLS for categorical predictions. Furthermore, multiple classifiers maybe invoked in step 254.

In step 260, a merging of results is performed. As indicated by the diamond-shaped symbols 256, multiple of the bagging classifiers may exist.

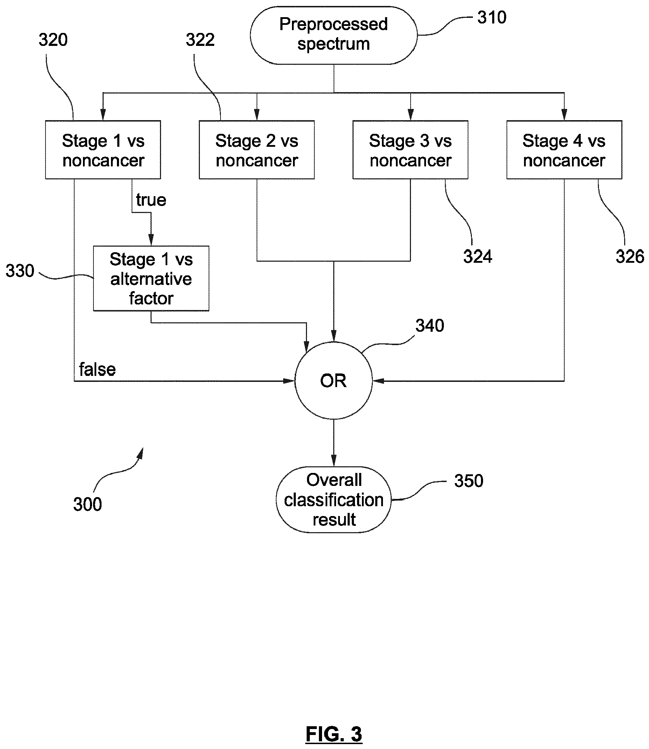

Steps 250 and 260 are illustrated in more detail in FIG. 3.

Since the spectrograms of different stages of lung cancer are quite different, a classifier may be built for each stage of lung cancer. This may include providing the pre-processed spectrum 310 that is obtained e.g. in step 240 to classifiers 320, 322, 324, 236 that classify stage 1 vs non-cancer (also referred to as control), stage 2 vs. non-cancer, stage 3 vs. non-cancer and stage 4 vs. non-cancer.

Certain stages of lung cancer, e.g. stage 1 lung cancer, are not clearly distinguishable from an alternative factor (also referred to as alternative state, and may be e.g. COPD or other lung diseases) as those can be more similar to stage 1 lung cancer than to the overall non-cancer group. But the alternative factor can still be distinguished from lung cancer in a direct comparison. As such, a second step 330 is added after the stage 1 classifier 320 in case the stage 1 classifier is positive. The second step 330 may be expanded to include several more models comparing a given cancer stage with one selected disease group. It applies to other alternative factors (non-cancer diseases such as COPD or other lung diseases) very similar to the specific cancer stage from the biochemical perspective. The general purpose of introducing the second step 330 to the algorithm architecture is to reduce the false-positive rate and increase the test correct classification rate. Overall, if any cancer stage classifier is positive, it is assumed that the patient associated with the sample has lung cancer. The binary OR operator 340 in the flowchart does exactly that, in order to obtain the overall classification result 350.

In the method of FIG. 3, each classifier's output may be binary (e.g. based on a 50% threshold). True may refer to a diagnosis of the disease (e.g. cancer), false may refer to the non-cancer group.

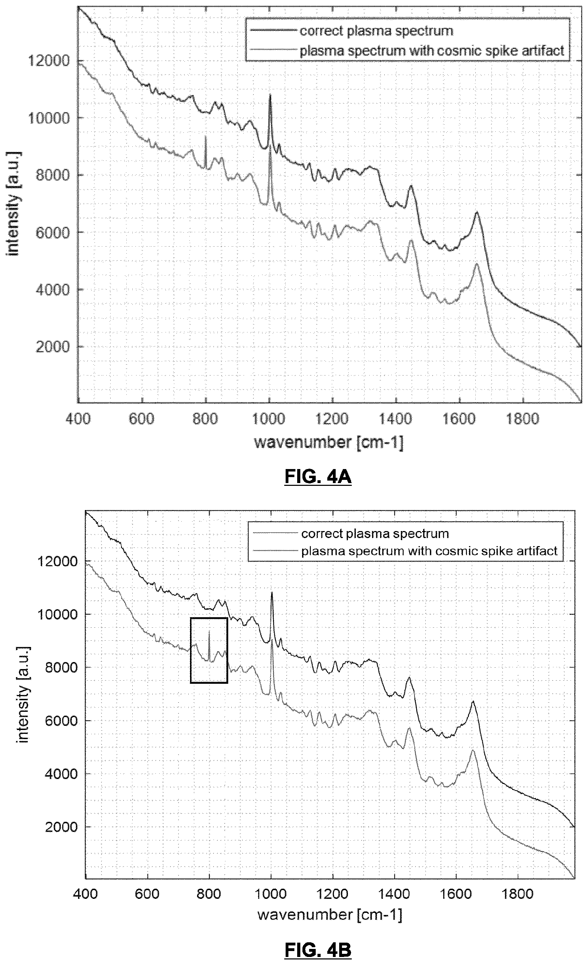

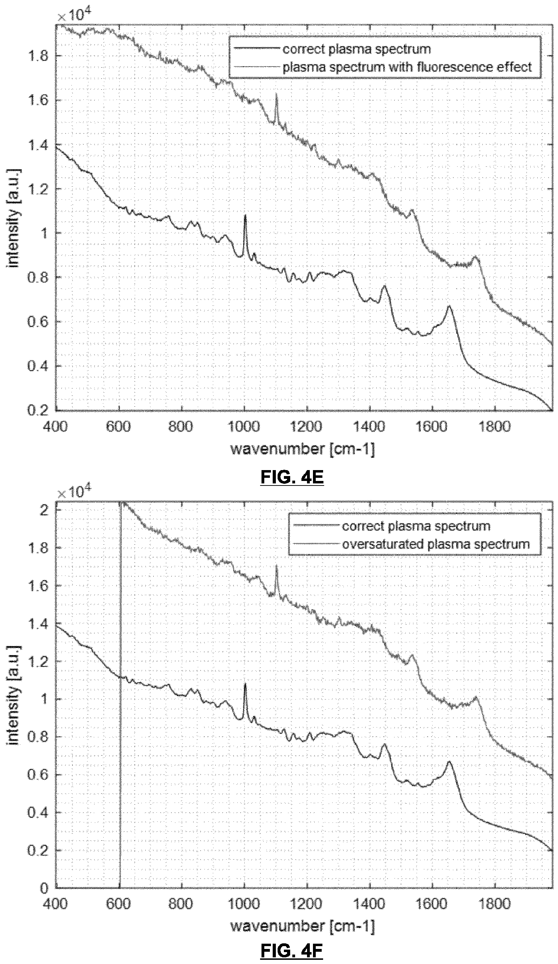

FIGs. 4A to 4E illustrate five different spectroscopic measurement data, each comprising two spectra. In FIG. 4A, the upper spectrum displays a correct plasma spectrum. The lower spectrum shows a spectrum that contains a cosmic spike, which is highlighted by the box around it and is rejected by the first outlier removal of the method illustrated in FIG. 3.

In FIG. 4B, the upper spectrum displays a correct plasma spectrum. The lower spectrum shows a spectrum that does not contain any spectral information that originates from blood plasma, but rather from spectrometer components, e.g. the objective lens, and is rejected by the second outlier removal.

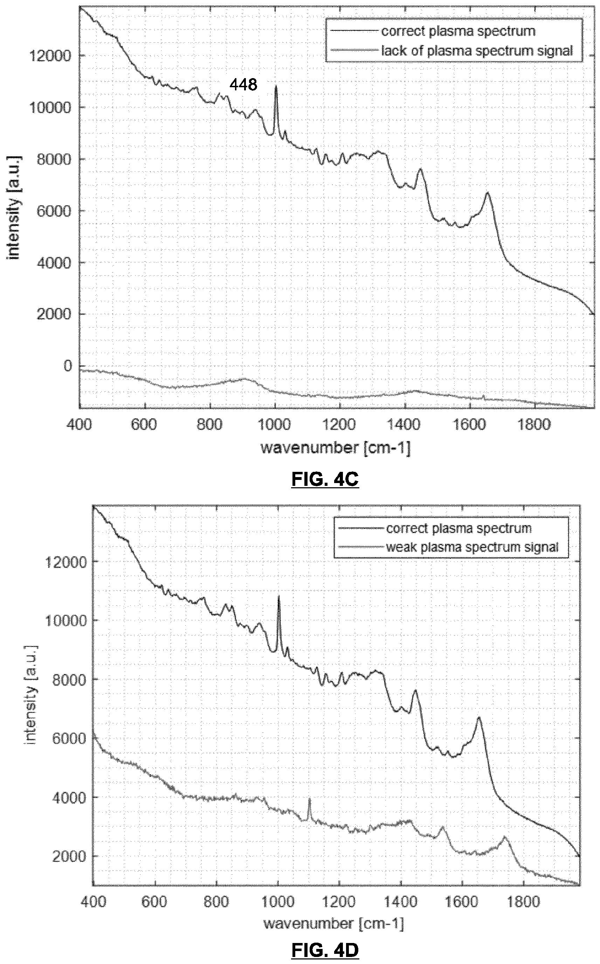

In FIG. 4C, the upper spectrum displays a correct plasma spectrum. The lower spectrum shows a spectrum that contains spectral information that originates from blood plasma, but would be considered "weak" and might contain additional spectral information from spectrometer components, e.g. the objective lens, and is rejected by the second outlier removal.

In FIG. 4D, the lower spectrum displays a correct plasma spectrum. The upper spectrum shows a spectrum that has a high level of fluorescence, which could originate from several sources, e.g. bacterial contamination and is rejected by the second outlier removal.

In FIG. 4E, the lower spectrum displays a correct plasma spectrum. The upper spectrum shows a spectrum that is oversaturated in a part of the spectrum that is relevant for data analysis (before 600 cm⁻¹) and is rejected by the second outlier removal.

The foregoing descriptions are only implementation manners of the present invention, the scope of the present invention is not limited to this. Any variations or replacements can be easily made through a person skilled in the art. Therefore, the protection scope of the present invention should be subject to the protection scope of the attached claims.

## Claims

1. A Computer-implemented method (100, 200) for diagnosing a disease in a subject, the method comprising:
- obtaining (110) spectroscopic measurement data comprising a plurality of spectra (420-448) of a sample of the subject;
- rejecting (120, 221) a spectrum from the plurality of spectra if the spectrum fulfils one or more spectrum outlier criteria;
- classifying (130, 250) the plurality of spectra using a plurality of classifiers (256, 320-330) to obtain a plurality of classification results; and
- determining (140, 260) a result indicative of whether the disease is present based on the plurality of classification results.

2. The method (100, 200) of claim 1, wherein the disease is a cancer, in particular a lung cancer.

3. The method (100, 200) of one of the previous claims, wherein the one or more spectrum outlier criteria comprise a spike value criterion that rejects a spectrum (420-448) if the spectrum comprises one or more values that exceed a median absolute variation of the spectrum.

4. The method (100, 200) of claim 3, wherein the median absolute variation is calculated based on a comparison of the plurality of spectra (420-448) using Chebyshev distance.

5. The method (100, 200) of one of the previous claims, wherein the plurality of classifiers (256, 320-330) comprise:
- one or more disease classifiers (320-326) that indicate whether the disease is present, and
- one or more alternate classifiers (330) that indicate whether one or more alternate states are present,
wherein in particular the one or more alternate states comprise chronic obstructive pulmonary disease, COPD and/or asthma.

6. The method (100, 200) of claim 5, wherein the disease is diagnosed if a disease confidence value of the one or more disease classifiers (320-326) is higher than an alternate confidence value of the one or more alternate classifiers.

7. The method (100, 200) of one of the previous claims, further comprising a step of rejecting (224) the plurality of spectra (420-448) based on one or more conditions of a class modelling technique that has been derived based on partial least square modelling of a training set of representative training spectra.

8. The method (100, 200) of one of the previous claims, wherein at least one of the plurality of classifiers (256, 320-330) is based on partial least-squares discriminant analysis.

9. The method (100, 200) of one of the previous claims, wherein the plurality of classifiers (256, 320-330) comprises a plurality of staging classifiers (320-326) that classify different stages of the disease against a control.

10. The method (100, 200) of claim 9, wherein the result indicates that the disease is present if at least one of the plurality of staging classifiers (320-326) yields a positive result.

11. The method (100, 200) of one of the previous claims, further comprising a step of normalizing (240) the plurality of spectra (420-448) by subtracting a mean of values of the plurality of spectra and dividing by a standard deviation of values of the plurality of spectra.

12. The method (100, 200) of one of the previous claims, wherein at least one of the plurality of classifiers (256, 320-330) comprises a bagging classifier that comprises a plurality of individual classifiers.

13. The method of one of claims 2 to 12, wherein the plurality of classifiers (256, 320-330) comprises classifiers for at least two different stages of the cancer, wherein preferably a positive result indicative of that a disease is present if at least one output of the classifiers for the at least two different stages of the cancer is positive.

14. An apparatus configured to carry out the method (100, 200) of one of the previous claims.

15. A computer-readable storage medium storing program code, the program code comprising instructions that when executed by a processor carry out the method (100, 200) of one of claims 1 to 13.
